# EUROPÄISCHE PATENTSCHRIFT

(11) **EP 0 699 906 B1**
(45) Veröffentlichungstag und Bekanntmachung des Hinweises auf die Patenterteilung: **24.04.2002**
(21) Anmeldenummer: 95111323.2
(22) Anmeldetag: 19.07.1995
(51) Int. Cl.: G01N 33/543, G01N 33/558, G01N 33/58, G01N 33/94

(54) **Verfahren zum Nachweis der Kontamination einer Oberfläche mit einem Analyten**
Method for detecting the contamination of a surface with an analyte
Méthode de détecter la contamination d'une surface avec une analyte

(30) Priorität: 25.07.1994 DE 4426281; 04.11.1994 DE 4439429
(43) Veröffentlichungstag der Anmeldung: 06.03.1996
(73) Patentinhaber: Roche Diagnostics GmbH, 68298 Mannheim (DE); SECURETEC GmbH, 85521 Ottobrunn (DE)
(72) Erfinder: Klein, Christian, Dr., D-82362 Weilheim (DE); Josel, Hans-Peter, Dr., D-82362 Weilheim (DE); Goerlach-Graw, Ada, Dr., D-67229 Grosskarlbach (DE); Hilpert, Reinhold, Dr., D-82272 Moorenweis (DE); Binder, Florian, Dr., D-83271 Traunstein (DE); Ritter, Josef, Dr., D-80339 München (DE); Zimmermann, Rudolf, D-83620 Vagen (DE)

(56) Entgegenhaltungen:
- EP-A- 0 262 328
- EP-A- 0 339 450
- EP-A- 0 436 897
- EP-A- 0 440 350
- SECURITY MANAGEMENT, Bd. 37, Nr. 8, 1993, Seiten 12-15, XP002006879 ADDIS ET AL.: "It's not a boy"

## Beschreibung

Die Erfindung betrifft ein Verfahren zum Nachweis der Kontamination einer Oberfläche durch einen Analyten durch Abwischen des Analyten von der Oberfläche mit einer Wischfläche, Elution des Analyten von der Wischfläche und Nachweis des Analyten in der Elutionsflüssigkeit durch eine immunologische Nachweisreaktion. Ferner betrifft die Erfindung ein entsprechendes Analyseelement.

Neben dem Nachweis von Analyten in Probeflüssigkeiten, wie z. B. Blut, Urin, Speichel, kommt insbesondere in der Kriminalistik dem Nachweis von Analyten auf festen Oberflächen, wie z. B. auf Möbeln, Gepäck usw., eine wachsende Bedeutung zu. Speziell in der Drogenfahndung ist es ein wünschenswertes Ziel, auch sehr geringe Drogenkontaminationen von Gegenständen einfach und schnell nachweisen zu können. Je sensitiver dabei ein Nachweisverfahren ist, um so weniger Analytmenge, die eine bestimmte Oberfläche kontaminiert, muß dabei auf dieser Oberfläche erfaßt werden.

Um beliebige Oberflächen auf Analyte, insbesondere auf Drogen, zu untersuchen, sind verschiedene spezielle Nachweistechniken bekannt, bei denen zuerst der Analyt durch Abwischen einer Oberfläche gesammelt und nach Elution von der benutzten Wischoberfläche immunologisch nachgewiesen wird. Bei dem "Illicit Substance Detector" der Firma Westinghouse, Baltimore, USA (Security Management, Vol. 37/8, Seite 12 - 15, 1993), wird mit einer genoppten Kunststoffoberfläche eine zu untersuchende Oberfläche abgewischt und die Droge über ein in eine Einwegkarte integriertes Verfahren mit drei Reagenzlösungen nachgewiesen (DE-A-4341862). Das Testergebnis wird mit einem optischen Lesegerät ausgewertet. Das immunologische Nachweisprinzip beruht auf der Inhibierung einer Latex-Agglutinationsreaktion durch die nachzuweisende Droge. Die untere Nachweisgrenze wird mit einem Mikrogramm angegeben. Nachteilig neben der recht hohen Nachweisgrenze ist, daß die Nachweisreaktion durch ein mechanisches Auspressen der drei Flüssigkeitsreservoirs gestartet werden muß. Zudem kann das Meßergebnis nur mit einer optischen Lesehilfe und nicht mit dem Auge ausgewertet werden.

Die Firma Roche Diagnostics stellt einen Testkit auf Kokain aus Urinproben nach demselben aufwendig zu handhabenden immunologischen Nachweisprinzip her. Die Nachweisempfindlichkeit liegt im Bereich von 0,2 µg/ml.

Der "Accupress-Kit" der Firma Thermetics, Woburn, USA (Security Management, Vol. 37/8, Seite 12 - 15, 1993), besteht aus einem speziell beschichteten Reagenzträger und drei Gefäßen mit Reagenzlösung. Bei diesem Test wird mit einem Wattestäbchen die zu untersuchende Oberfläche abgewischt und das Wattestäbchen anschließend mit Puffer ausgewaschen. Neben der vergleichbar unempfindlichen Nachweisgrenze von nicht unter einem µg ist die Handhabung von drei verschiedenen Reagenzlösungen umständlich und zieht auch Fehlermöglichkeiten nach sich.

Aus EP-A 0 262 328 ist bekannt, daß immunologische Nachweisreaktionen für Analyte in Flüssigkeiten mittels mehrzoniger Chromatographie-Teststreifen durchgeführt werden können. Die für die Nachweisreaktionen erforderlichen Reagenzien (u. a. Bindepartner für den Analyten, Markierungssubstanzen) können im Teststreifen in löslicher und immobilisierter Form in den unterschiedlichen Zonen vorliegen.

Aufgabe der Erfindung war es daher, ein empfindlicheres Verfahren zum Nachweis von Analytkontaminationen von Oberflächen, insbesondere mit Drogenspuren, bereitzustellen, das in einfacher Weise und ohne technische Hilfsmittel durchgeführt werden kann. Insbesondere sollte die Nachweisgrenze für den Analyt deutlich unter einem µg absolut, möglichst unter 100 ng liegen.

Gelöst wird die Aufgabe durch ein Verfahren und ein Analysenelement, wie es in den Ansprüchen charakterisiert ist.

Gegenstand der Erfindung ist ein Verfahren zum Nachweis der Kontamination einer Oberfläche durch einen Analyten durch Abwischen des Analyten von der Oberfläche mit einer Wischfläche eines Wischmaterials, Elution des Analyten von der Wischfläche und Nachweis des Analyten in der Elutionsflüssigkeit durch eine immunologische Nachweisreaktion, dadurch gekennzeichnet, daß
a) mit der Wischfläche die auf den Analyt zu untersuchende Oberfläche abgewischt wird,
b) die Wischfläche mit der flächigen Oberfläche eines kapillaraktiven chromatographiefähigen Teststreifens, der eine Elutionsmittelaufgabezone an einem Ende und eine Zielzone am anderen Ende des Streifens enthält, in einem Bereich zwischen beiden Zonen kontaktiert wird,
c) Elutionsflüssigkeit auf die Elutionsmittelaufgabezone aufgegeben wird, die durch Kapillarkraft zur Zielzone an der Kontaktstelle mit der Wischfläche vorbeiwandert, wobei Analyt auf der Wischfläche von der Elutionsflüssigkeit aufgenommen wird und
d) der Analyt in der Zielzone aufgrund einer immunologischen Bindungsreaktion gemessen wird.

Der Teststreifen für das erfindungsgemäße Verfahren kann aus einem einzigen chromatographiefähigen streifenförmigen Material oder bevorzugt aus mehreren auf einer Basisschicht im wesentlichen nebeneinander angeordneten kapillaraktiven Flächen aus gleichen oder verschiedenen Materialien bestehen, die in Fluidkontakt zueinander stehen, so daß sie eine Flüssigkeitstransportstrecke bilden, entlang derer eine Flüssigkeit, durch Kapillarkräfte getrieben, von der Elutionsmittelaufgabezone zu der Zielzone strömt.

Als chromatographisches Material kommen alle flüssigkeitsabsorbierenden, porösen oder kapillaraktiven Materialien in Frage, z.B. Cellulose und deren Derivate, Glasfasern und Vliese und Gewebe aus künstlichen oder natürlichen Materialien.

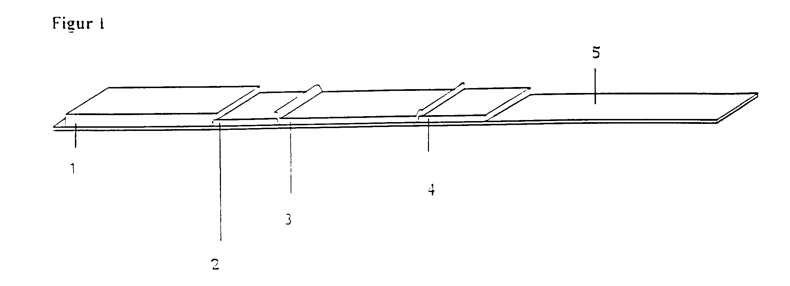

Es können in dem erfindungsgemäßen Verfahren verschiedene immunologische Testführungen angewendet werden mit denen der Analyt aufgrund einer oder mehrerer immunologischer Bindungsreaktionen nachgewiesen wird. In einer bevorzugten Ausführungsform (Figur 1) enthält ein für die Erfindung geeigneter Chromatographie-Teststreifen gegebenenfalls auf einer Trägerfolie (5) zwischen der Elutionsmittelaufgabezone (1) und der Zielzone (4) eine Abfangzone (3), die ein Abfangreagenz in immobilisierter Form enthält, das fähig ist, entweder den Analyten, einen spezifischen Bindungspartner des Analyten oder einen markierten Bindungspartner spezifisch zu binden. Vor der Abfangzone enthält der Teststreifen bevorzugt eine Konjugatzone (2), die einen wanderungsfähigen markierten Bindungspartner enthält, der fähig ist, entweder den Analyten, einen spezifischen Bindungspartner des Analyten oder das Abfangreagenz in der Abfangzone spezifisch zu binden.

Bei dem "spezifischen Bindungspartner des Analyten" handelt es sich um eine wanderungsfähigen, unmarkierten Analytbindungspartner mit einer Bindungsstelle für das Abfangreagenz. Wenn ein solcher Bindungspartner in dem Immunoassay verwendet wird, so kann dieser vor oder in der Konjugatzone oder bevorzugt zwischen Konjugatzone und Abfangzone aufgebracht sein. Zusätzlich kann sich nach der Zielzone noch weiteres flüssigkeitsabsorbierendes Material anschließen, das Flüssigkeit nach einer Wanderung durch die einzelnen Zonen aufnimmt.

Je nach Art des Immunoassays, der in dem erfindungsgemäßen Verfahren angewandt werden soll, liegen in den verschiedenen Zonen unterschiedliche Bindungspartner vor: Bei einem Sandwich-Immunoassay liegen bevorzugt in der Konjugatzone ein markierter Analytbindungspartner nicht-immobilisiert vor. Dieser bildet mit dem Analyten einen Komplex, der entweder durch das Abfangreagenz durch dessen Bindung an den Analyten gebunden wird, oder ein zweiter, unmarkierter, frei wanderungsfähiger Analytbindepartner mit einer spezifischen Bindungsstelle für das Abfangreagenz bildet zuerst einen Sandwich-Komplex mit dem Analyten, der mit Hilfe der spezifischen Bindungsstelle des Bindungspartners in der Abfangzone gebunden wird. Bevorzugt wird dabei die Markierung des Komplexes in der Abfangzone gemessen. In diesem Fall sind Abfangzone und Zielzone identisch.

Bei einem kompetitiven Test befindet sich bevorzugt in der Konjugatzone ein markiertes Analyt-Analogon, das bei Anwesenheit des Analyten entweder um die Bindungsstellen des Abfangreagenzes in der Abfangzone konkurriert (in diesem Fall ist das Abfangreagenz ein Analytbindungspartner) oder, wenn ein zusätzlicher wanderungsfähiger Analytbindungspartner vorhanden ist, um dessen Bindungsstelle konkurriert. Komplexe von markiertem Analyt-Analogon und wanderungsfähigem Bindungspartner werden dann über eine spezifische Bindungsstelle, z. B. Biotin, in der Abfangzone gebunden. In diesem Falle ist das Abfangreagenz ein Bindungspartner des wanderungsfähigen Analytbindungspartners, z. B. Streptavidin. Bevorzugt wird bei diesem Verfahren die Markierung nicht in der Abfangzone, sondern in der Zielzone in Form der unkomplexierten Analyt-Analoga als Maß für die Anwesenheit des Analyten gemessen.

Bevorzugt wird das erfindungsgemäße Verfahren nach einem IEMA-analogen Testprinzip (auch "immunenzymometrisch-analoges" Testprinzip) durchgeführt. Die Durchführung von IEMA-Tests auf Teststreifen ist beispielsweise in EP-A-0 407 904, EP-A-0 353 570 oder DE OS 4024919 beschrieben.

In der Konjugatzone befinden sich markierte Bindungspartner für den Analyten im Überschuß. Nach chromatographischer Wanderung werden nicht an Analyt gebundene markierte Bindungspartner durch festphasengebundene Analyt-Analoga in der Abfangzone immobilisiert, während Komplexe aus Analyt und markiertem Bindungspartner in die Zielzone weiterchromatographieren. Die in die Zielzone gelangte Markierung kann als Maß für die Anwesenheit oder auch die Konzentration des Analyten gemessen werden.

Als Analyt dienen in der vorliegenden Erfindung alle immunologisch nachweisbaren Substanzen insbesondere Antigene und Haptene. Ganz besonders geeignet ist die vorliegende Erfindung zum Nachweis von Drogen, z. B. Kokain, Morphin, Heroin. Dabei kann der Analyt auf der abzuwischenden Oberfläche als Molekül oder partikulär oder an Partikel adsorbiert vorkommen.

Als Markierung kommen übliche Markierungen, wie Enzymmarkierung, Fluoreszenz-, Farbstoffmarkierung, in Frage. Bevorzugt ist Direktmarkierung, insbesondere Metallmarkierung, ganz besonders bevorzugt Goldmarkierung. Diese hat den Vorteil, daß direkt mit dem Auge das Testergebnis abgelesen werden kann.

Als Bindungspartner für den Analyten kommen insbesondere Antikörper und Antikörperfragmente in Betracht.

Falls ein wanderungsfähiger unmarkierter Analytbindungspartner zusätzlich zum Abfangreagenz benutzt wird, trägt dieser eine Bindungsstelle für das Abfangreagenz. Für diese Bindungsstelle kommen alle spezifischen Bindungspartner eines spezifischen Bindungspaares in Betracht, z. B. Lektine, Antikörper, Antigene, bevorzugt Biotin (das mit Streptavidin bindet). Die Markierung in der Abfangzone bzw. Zielzone kann über übliche Nachweismethoden erfolgen, z. B. reflektionsphotometrisch oder visuell. Insbesondere bei Metallmarkierung, z. B. Goldmarkierung, kann das Meßergebnis in einfacher Weise visuell abgelesen werden.

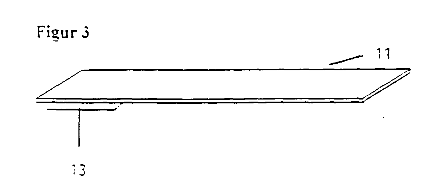

Zur Durchführung des erfindungsgemäßen Verfahrens wird mit einer Wischoberfläche über eine zu untersuchende Oberfläche vorteilhafterweise unter Anwendung eines leichten Drucks gewischt. Die zu untersuchende Oberfläche kann dabei trocken oder mit einem analythaltigen Film bedeckt sein. Es ist vorteilhaft, wenn der Wischvorgang mehrmals wiederholt wird. Zur besseren Handhabung ist die Wischoberfläche (13) bevorzugt auf einem Träger (11) befestigt (Figur 3). Eine gute Wischleistung ergibt sich insbesondere bei einer hohen mechanischen Beanspruchbarkeit der Wischoberfläche. Besonders vorteilhaft hat es sich dafür erwiesen, wenn das Material der Wischfläche mit dem Träger (11) mit Ultraschall verschweißt wird.

Als Wischfläche können alle Materialien wie Kunststoffe, Gewebe, Vliese benutzt werden, auf denen Analyte, insbesondere Drogen haften bleiben und sich durch Wischen anreichern lassen.

Andererseits sollten die anhaftenden Analyten bei Flüssigkeitskontakt wieder leicht desorbierbar sein. Der Fachmann kann ohne Aufwand solche Materialien auswählen. Bevorzugt sind saugfähige Materialien wie Vliese, Gewebe oder poröse Matrices wie Membrane oder Schwämme. Ganz besonders geeignet sind Vliese aus faserigen Materialien, wobei die Fasern im allgemeinen ungeordnet zusammengefügt sind, z.B. Papier oder Glasfaservliese. Die Wischoberfläche ist bevorzugt trocken, kann aber auch angefeuchtet werden.

Werden rauhe Oberflächen wie z.B. anodisierte Aluminiumoberflächen oder andere angerauhte Metalloberflächen untersucht, wird bevorzugt eine angefeuchtete Wischoberfläche eingesetzt. Geeignete Flüssigkeiten zur Anfeuchtung des Wischfläche sind primär Wasser und wäßrige Puffersysteme, die auf die nachfolgende Immunreaktion abgestimmt sein können. Dem Wasser oder den wäßrigen Puffern können Detergentien oder organische Lösungsmittel zugefügt sein. Als Detergentien können Tween 20, Tween 80, Octylglucosid, Polidocanol, Synperonic, z.B. F 68, oder auch zwitterionische Detergentien wie Cholamidopropansulfonat in einer Konzentration unter 5 Gew.- %, bevorzugt bei 0,01 Gew.-% bis 1 Gew.-% allein oder in Mischungen verwendet werden. Als organische Lösungsmittel können z.B. Dimethylsulfoxid, Glycerin oder Ethanol allein oder in Mischungen eingesetzt werden. Der Anteil an Lösungsmittel in der Flüssigkeit kann deutlich unter 30 Gew.-% betragen. Es ist aber auch möglich, organische Lösemittel oder Mischungen organischer Lösungsmittel ohne Wasser zu verwenden.

Die Befeuchtung der Wischfläche wird durch die Aufgabe von 1 - 50 µl der Flüssigkeit pro cm², bevorzugt 3 - 20 µl/cm² z. B. durch Aufgabe mit einer Pipette oder einer automatischen Dosiervorrichtung erreicht. Alternativ kann die Befeuchtung auch durch kurzzeitigen Kontakt der Wischfläche mit einem feuchten Schwamm oder einer anderen feuchtigkeitsabgebenden Oberfläche erfolgen.

Die Befeuchtung der Wischfläche kann auch vorteilhaft so gelöst werden, daß die Flüssigkeit in mikroverkapselter oder blisterverpackter Form auf dem für das Wischen vorgesehenen Teil bereits vorgehalten wird. Wenn als Flüssigkeit Wasser oder wässrige Puffersysteme eingesetzt werden, wird die Verkapselung vorteilhaft durch Einschluß in wachsartige Substanzen, wie z.B. Paraffin, vorgenommen. Das Freisetzen der Flüssigkeit kann auf mechanischem Weg, z.B. durch Andrücken auf der zu untersuchenden Oberfläche, erfolgen.

Bevorzugt ist die Wischfläche ein Vlies bevorzugt aus Fasern auf der Basis von Zellulose und / oder Polyesterfasern. Zusätzlich können die Fasern von einem organischen Bindemittel, das bevorzugt Hydroxyl und/oder Estergruppen enthält, zusammengehalten werden. Solche Vliese sind in der deutschen Patentanmeldung DE-OS-3802366 beschrieben.

Bevorzugt werden als Zellulosefasern Zellwolle, Zellstoff oder Linters eingesetzt. Als Zellwolle wird ein Material bezeichnet, das durch Alkalisieren von Zellulose zu Alkali-Zellulose, anschließender Behandlung mit Schwefelkohlenstoff und der Bildung von Zellulose-Xanthogenaten, Auflösung der Zellulose-Xanthogenate in Lauge und Verspinnen von Viskosefilamentgarn erhalten wurde. Zellstoff kann durch einen vollständigen chemischen Aufschluß zellulosehaltiger Materialien und anschließende Bleiche gewonnen werden. Als Linters werden kurze, nicht spinnbare Baumwollfasern bezeichnet, die aus Baumwollsamen erhalten werden. Die Zellulosefasern weisen bevorzugt eine Faserfeinheit von 1,7 bis 4,5 dtex auf und besitzen Längen von 1 bis 20 mm, bevorzugt 3 bis 12 mm. Besonders bevorzugte Polyesterfasern sind Fasern mit einem spezifischen Gewicht von ca. 1,17 g/cm³, einer Länge von 3 bis 6 mm und einer Faserfeinheit von 1,7 bis 3,3 dtex.

Als weiteren Bestandteil können die Vliese ein organisches Bindemittel, das OH- und/oder Estergruppen aufweist, enthalten. Bevorzugt werden hierzu Polyvinylalkohol und Epichlorhydrinharze eingesetzt. Der Polyvinylalkohol wird vorzugsweise als Fasermaterial mit einer Länge von 3-5 mm und einem spezifischen Gewicht von 1,26 bis 1,30 g/cm³ eingesetzt. Aus diesen Komponenten und voll entsalztem Wasser wird ein Vlies auf einer Schrägsiebmaschine nach dem üblichen Verfahren der Papierherstellung erzeugt. Besonders bevorzugte Vliesmaterialien sind außerdem in DE-OS-3802366 beschrieben.

Die Dicke des Materials der Wischfläche ist nicht entscheidend. Vorteilhafterweise sollte es eine möglichst ebene Oberfläche haben, wobei die Dicke üblicherweise zwischen 0,1 und 3 mm liegt. Die Wischoberfläche sollte an die Dimensionen des Chromatographie-Teststreifens angepaßt sein, d.h. die Breite der Wischfläche sollte die Breite des Teststreifens nicht wesentlich übersteigen. Bevorzugte Dimensionen der Wischfläche liegen zwischen 0,3 und 2 cm, besonders bevorzugt von 0,5 bis 0,8 cm in der Länge und in der Breite von 0,3 bis 1 cm, besonders bevorzugt von 0,4 bis 0,8 cm.

Nach dem Abwischen einer kontaminierten Oberfläche mit einer Wischfläche wird diese Wischoberfläche mit einem Bereich der Teststreifenoberfläche zwischen der Elutionsmittelaufgabezone und der Zielzone kontaktiert, bevorzugt leicht aufgedrückt.. Dieser Bereich liegt bevorzugt zwischen der Elutionsmittelaufgabezone und der Konjugatzone oder auf der Konjugatzone selbst. Besonders bevorzugt ist außerdem, wenn die Zone, auf der die Wischfläche aufgedrückt wird, aus einem der für die Wischfläche geeigneten Materialien besteht, insbesondere den in der DE-OS-3802366 beschriebenen Vliesmaterialien. Ganz besonders bevorzugt ist es, wenn die Wischfläche auf die Konjugatzone aufgedrückt wird. Bevorzugt ist deshalb auch, daß die Wischfläche zwischen 25% und 150% der Fläche, besonders bevorzugt annähernd die gleiche Fläche wie die Konjugatzone hat.

Der Druck, mit dem die Wischfläche aufgedrückt wird, sollte mindestens so groß sein, daß ein flächiger Fluidkontakt zwischen beiden Oberflächen möglich ist.

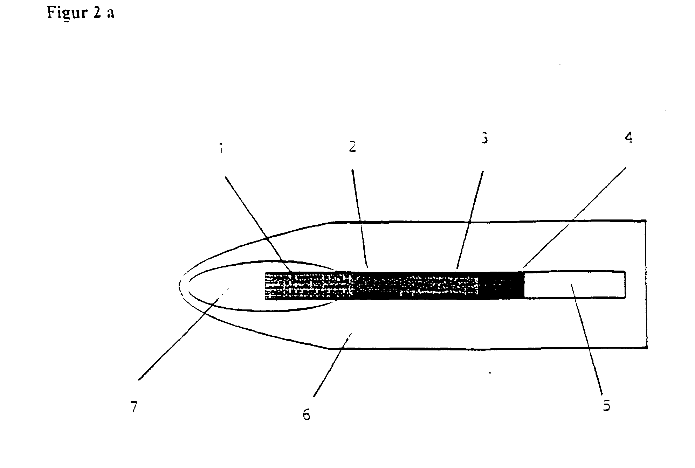

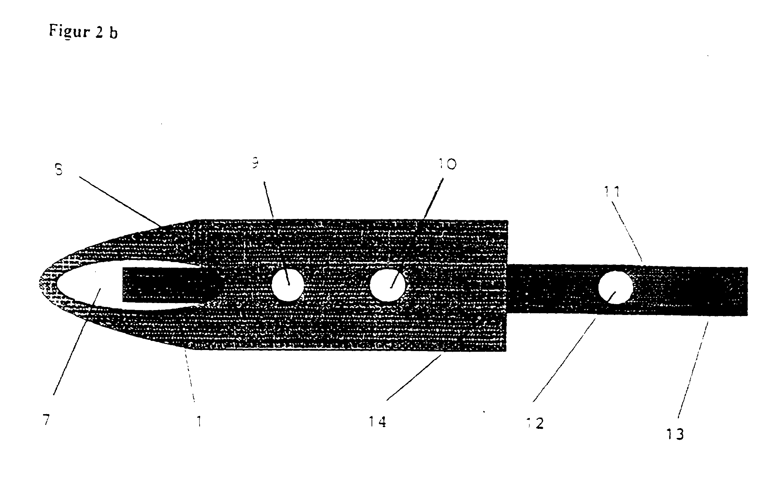

Um dem Benutzer das Aufdrücken zu erleichtern, kann in einer Ausführungsform der Testträger (5) in einem Gehäuse (6,8) untergebracht sein (Figur 2a: Gehäuse ohne Deckel, Figur 2b: Gehäuse geschlossen). Das Gehäuse weist eine Öffnung (9) zum Aufdrücken der Wischfläche (13) auf. Die Wischfläche (13) ist auf einem Träger (11) so an ein Scharnier (14) des Gehäuses befestigt, daß dieser Träger zum Wischen ausgeklappt ist und zum Aufdrücken der Wischfläche auf die Öffnung (9) des Teststreifengehäuses umgeklappt und gegebenenfalls arretiert werden kann. Die Wischflächee kann auch mit der Hand oder einer Klammer mit dem Teststreifen kontaktiert werden.

Im nächsten Schritt wird Elutionsflüssigkeit auf die Elutionsmittelaufgabezone (1) aufgebracht. Die Flüssigkeit kann auf die Elutionsmittelaufgabezone aufgegeben werden oder der Teststreifen in eine Elutionsflüssigkeit getaucht werden. Wird die Flüssigkeit aufgebracht, besteht die Elutionsmittelaufnahmezone bevorzugt aus einem besonders saugfähigen Material, das mindestens so viel Flüssigkeit aufnimmt, daß die Flüssigkeit bis zum Ende des Chromatographie-Streifens wandert. Ist der Testträger in einem Gehäuse, hat das Gehäuse bevorzugt eine Öffnung (7) zur Aufgabe der Flüssigkeit.

Als Elutionsflüssigkeit kommt Wasser und die bei Immunoassays üblichen Pufferlösungen in Frage. Die Flüssigkeit wandert entlang des Streifens in Richtung Zielzone (4) und passiert dabei die Zone mit der aufgedrückten Wischfläche. Überraschenderweise werden dabei auf der Wischfläche haftende Analytmoleküle von dem Flüssigkeitsstrom aufgenommen und in die weiteren Zonen weitertransportiert. Bevorzugt ist, wenn die Zone des Teststreifens, auf die die Wischfläche aufgedrückt wird ("Aufnahmezone") aus einem der für die Wischfläche bevorzugten Vlies-Materialien besteht. Besonders bevorzugt ist, wenn die Aufnahmezone ("Aufnahmevlies") durch die Konjugatzone (2) selbst gebildet wird.

In einer bevorzugten Testvariante werden Analytmoleküle beim Passieren der Konjugatzone durch markierte Analyt-Bindungspartner komplexiert, nicht-komplexierte markierte Bindungspartner werden in der Abfangzone durch immobilisierte Analyt-Analoga, z. B. Polyhaptene, festgehalten, während markierte Bindungspartner, die einen Analyten gebunden haben, die Abfangzone passieren und die Zielzone erreichen. In der Zielzone können dann die markierten Komplexe detektiert werden. Um das Signal in der Zielzone optisch besser von der Markierung in der Abfangzone unterscheiden zu können, kann die Abfangzone vorteilhafterweise abgedeckt sein. Wenn der Testträger in einem Gehäuse untergebracht ist, hat dieses über der Zielzone bevorzugt eine Öffnung (10) zum Beobachten des Signals.

Die Analyse auf eine Analytkontamination der untersuchten Oberfläche ist dann positiv, wenn mindestens ein Teilbereich der Zielzone eine Färbung aufweist. Die Färbung kann leicht visuell oder photometrisch detektiert werden.

Es ist auch möglich, mit einem Teststreifendevice mehrere Analyten nachzuweisen. Hierzu kann die Konjugatzone und die Abfangzone und gegebenenfalls auch das chromatographische Material von der Konjugatzone bis zur Zielzone in mehrere, in Teststreifenrichtung parallele, vorteilhafterweise voneinander getrennte Teilstreifen unterteilt sein, wobei in einzelnen Teilkonjugatzonen und Teilabfangzonen Bindungspartner für die unterschiedlichen nachzuweisenden Analyten oder unterschiedliche Analytanaloga enthalten sind. Nach Kontaktieren der Wischfläche mit einer gemeinsamen Aufnahmezone vor den Konjugatzonen und Aufgabe der Elutionsflüssigkeit, teilt sich in den verschiedenen Teilkonjugatzonen die Analytflüssigkeit auf verschiedene Teilchromatographiestrecken auf, wobei in jeder Teilzielzone ein anderer Analyt nachgewiesen werden kann.

Die Empfindlichkeit des erfindungsgemäßen Verfahrens ist überraschenderweise wesentlich höher als die der Verfahren aus dem Stand der Technik. Die Wisch- und Übertragungseffizienz der Wischfläche von Analyten auf den Teststreifen ist wider Erwarten so groß, daß mit dem Verfahren Absolutmengen von bis zu 10 ng Analyt, insbesondere Drogen, auf Oberflächen nachgewiesen werden können. Das Verfahren erfordert sehr wenige Handhabungsschritte und das Ergebnis kann sehr schnell und mit einfachen Mitteln ermittelt werden.

Ein weiterer Gegenstand der Erfindung ist ein Testkit zum Nachweis der Kontamination einer Oberfläche mit einem Analyten durch eine immunologische Nachweisreaktion, dadurch gekennzeichnet, daß es umfaßt
a) einen Teststreifen aus einem oder mehreren kapillaraktiven chromatographiefähigen flächenförmigen Materialien in Fluidkontakt zueinander mit
   - einer Elutionsmittelaufgabezone an einem Ende und einer Zielzone am anderen Ende,
   - einer Abfangzone zwischen Elutionsmittelaufgabezone und Zielzone, in der ein Abfangreagenz immobilisiert ist, das fähig ist, entweder den Analyten, einen spezifischen Analytbindungspartner oder einen markierten Bindungspartner spezifisch zu binden und
   - einer Konjugatzone, zwischen Elutionsmittelaufgabezone und Abfangzone, die einen wanderungsfähigen markierten Bindungspartner enthält, der fähig ist, entweder den Analyten oder den spezifischen Analytbindungspartner oder das Abfangreagenz spezifisch zu binden, wobei die Bindung des markierten Bindungspartners zu einem detektierbaren Signal in der Abfangzone oder in der Zielzone führt, das die Anwesenheit des Analyten anzeigt,
b) eine Wischfläche separat zur Teststreifenoberfläche
c) eine Andrückvorrichtung, die bewirkt, daß die Wischfläche mit der Teststreifenoberfläche zwischen Elutionsmittelauftragszone und Konjugatzone oder bevorzugt in der Konjugatzone selbst kontaktiert werden kann.

### Beispiel 1

### a. Herstellung von Benzoylecgoninmaleimidoethylamid

2.4 g Benzoylecgoninhydrochlorid werden in 200 ml trockenem Acetonitril mit 1 g N-Hydroxysuccinimid und 1.8 g Dicyclohexylcarbodiimid versetzt und 3 Std. gerührt. Vom Niederschlag wird abfiltitriert, das Filtrat eingedampft, in Nitromethan aufgenommen und nochmals filtriert. Nach Abdampfen des Lösungsmittels wird mit Ether verrieben. Man erhält 1.13 g Benzoylecgoninsuccinimidylester. Dieses Produkt wird zusammen mit 0.47 g Maleimidoethylaminhydrochlorid (s. WO 90/15798) in 100 ml trockenem Acetonitril aufgenommen. Man gibt 1.1 g Triethylamin zu und rührt 12 Stunden bei Raumtemperatur. Das Reaktionsgemisch wird eingedampft, in 50 ml Essigester aufgenommen und drei Mal mit Natriumhydrogencarbonatlösung ausgeschüttelt. Die Essigesterphase wird eingedampft und das Produkt durch Aufnahme in 10 ml mit HCl gesättigtem Dioxan in das Hydrochlorid überführt. Man filtriert, wäscht mit Ether und erhält so 1 g Benzoylecgoninmaleimidoethylamidhydrochlorid.

### b. Herstellung eines biotinylierten Cocain-Polyhaptens für die Abfangzone

Kaninchen-IgG wird bei einer Konzentration von 25 mg/ml in Phospatpuffer pH 8 mit der 6fach molaren Menge S-Acetylthiopropionsäuresuccinimidylester gelöst in Dimethylsulfoxid umgesetzt. Nach 1 Stunde bei 25 °C wird die Reaktion durch Zugabe einer Lösung von 1 mol/l Lysin gestoppt. Es folgt Dialyse gegen 0.1 mol/l Kaliumphosphatpuffer, pH 6 mit 1 mmol/l EDTA. Anschließend wird der pH auf 7.8 eingestellt und mit 1 mol/l Hydroxylamin-Lösung, pH 7,5 ad 2o mmol/l 1 Stunde bei 25 °C inkubiert. Zur Kopplung wird ein 5fach molarer Überschuß Benzoylecgoninmaleimidoethylamidhydrochlorid in Dimethylsulfoxid gelöst und unter Rühren zu der Lösung des mit Sulfhydrylgruppen modifizierten Kaninchen-IgG gegeben. Nach Inkubation bei 25 °C für 2 Stunden wird die Reaktion gestoppt durch die successive Zugabe von 0.1 mol/l Cystein-Lösung ad 1 mmol/l und 0.5 mol/l Jodacetamidlösung ad 5 mmol/l. Der Ansatz wird über Nacht gegen 0.1 mol/l Kaliumphosphatpuffer, pH 8.5 dialysiert und über Membranfiltration auf eine Proteinkonzentration von 10 mg/ml konzentriert. Danach wird das erhaltene Cocain-Polyhapten mit einem 8fach molarem Überschuß Biotinylcapronsäuresuccinimidylester, gelöst in Dimethylsulfoxid, biotinyliert. Der Ansatz wird gegen 20 mmol/l Natriumacetat, pH 4.3, dialysiert und über FPLC aufgereinigt.

### c. Herstellung von Morphin-3-O-essigsäuremaleimidoethylamidhydrochlorid

Analog Beispiel 1 a. wird Morphin-3-O-essigsäure mit Maleimidoethylaminhydrochlorid zu Morphin-3-O-essigsäuremaleimidoethylamidhydrochlorid umgesetzt.

### d. Herstellung eines biotinylierten Morphin-Polyhaptenes

Analog Beispiel 1 b. wird mit Sulfhydrylgruppen modifiziertes Kaninchen-IgG mit Morphin-3-O-essigsäuremaleimidoethylamidhydrochlorid und Biotinylcapronsäuresuccinimidylester zu einem biotinylierten Morphin-Polyhapten umgesetzt.

### e. Herstellung eines Goldkonjugates aus einem Anti-Cocain-Antikörper

Goldsol mit einem durch Photonen-Korrelations-Spektroskopie bestimmten Partikeldurchmesser von 20 nm wurde nach Standardverfahren (Frens, Nature Vol. 241, S. 20-22, 1973) hergestellt. Die Konjugation mit dem Antikörper, der Cocain und Benzoylecgonin erkennt, wird nach dem Stand der Technik durchgeführt (Geoghegan et al., J. Immunol. Meth. Vol.. 34, S. 11-31, 1980)

### f. Herstellung eines Goldkonjugates aus einem Anti-Morphin-Antikörper

Analog Beispiel 1 e wurde ein Antikörper, der Morphin und Heroin erkennt, an Goldpartikel gebunden.

### Beispiel 2

### a. Testträger zur Bestimmung von Cocain

Aufbau des Testträgers siehe Figur 1.

### Elutionsmittelaufgabezone (Saugvlies) (1)

Polyestervlies der Firma Binzer, Hatzfeld, Bundesrepublik Deutschland. Es handelt sich um ein reines Polyestervlies, das mit 10% Kuralon verfestigt ist. Die Dicke beträgt 1,0 - 1,2 mm, die Saugkapazität 1800 ml/m²

### Konjugatzone (Konjugatvlies) (2)

Ein Mischvlies aus 80 Teilen Polyester und 20 Teilen Zellwolle, verfestigt mit 20 Teilen Kuralon, in einer Dicke von 0,32 mm und mit einer Saugkapazität von 500 ml/m² wird mit folgender Lösung getränkt und anschließend getrocknet: 100 mmol/l HEPES-Puffer pH 7.5, 100 mol/l NaCl, Konjugat aus Goldpartikeln und einem Anti-Cocain-Antikörper, der auch an Benzylecgonin bindet in einer Konzentration, die eine optische Dichte von 10 bei 520 nm aufweist.

### Abfangzone (3)

Ein Vlies aus 100% Linters, verfestigt mit 2% Etadurin mit einer Dicke von 0.35 mm und einer Saugkapazität von 372 ml/m² wird mit folgender Lösung getränkt und anschließend getrocknet: 10 mmol/l Natriumphosphat pH 7.5, polymerisiertes Streptavidin 200 mg/l (Herstellung nach Beispiel 1c, EP A 0 331 127). Das vorgetränkte Vlies wird anschließend nochmals getränkt und anschließend getrocknet: 10 mmol/l Natriumphosphat pH 7.5, 200 mg/l biotinyliertes Cocain-Polyhapten aus Beispiel 1 b.

### Nachweisfeld (Zielzone) (4)

Es wird ein Vlies aus 100% Linters, verfestigt mit 2% Etadurin mit einer Dicke von 0.35 mm und einer Saugkapazität von 372 ml/m² eingesetzt.

Alle Vliese haben eine Breite von 5 mm. Das Konjuagtvlies hat eine Größe von 5 x 5 mm. Die Vliese werden gemäß Fig. 1 auf eine Trägerfolie (5) von 5 mm Breite geklebt.

### b. Testträger zum Nachweis von Heroin

Analog wird aus dem Goldkonjugat des Anti-Heroin-Antikörpers und dem biotinylierten Morphin-Polyhapten ein Testträger zum Nachweis von Heroin hergestellt.

### Beispiel 3

Auf eine Polyethylenoberfläche werden jeweils 10 µl einer verdünnten Heroinhydrochloridlösung in Methanol aufgetragen und eintrocknen gelassen. Man erhält je mit 10, 20, 40, 60 und 80 ng Heroinhydrochlorid kontaminierte Flächen von ca. 1 cm² Fläche. Von jeder Menge werden drei Testfelder präpariert.

Es wurde ein Vlies hergestellt, das aus 80 Teilen Polyesterfasern einer Faserfeinheit von 3,3 dtex und einer Faserlänge von 4 mm besteht, 20 Teilen Zellwolle mit einer Faserfeinheit von 1,7 dtex und einer Schnittlänge von 3 mm sowie 20 Teilen Polyvinylalkoholfasern einer Schnittlänge von 4 mm. Die Faserstoffe Polyester, Zellwolle und Polyvinylalkohol wurden mit VE-Wasser bei einer Stoffdichte von 0,3% in Mischbütten aufgeschlagen bzw. vereinzelt. Der Faserstoff wurde anschließend auf ein umlaufendes Sieb gepumpt. Während das Fasergemisch entwässert bzw. das Wasser durch Vakuum abgesaugt wird, orientieren sich die Fasern auf der Siebseite und werden als Vlies mit einem Trockengehalt von ca. 20% über Trockenzylinder kontaktgetrocknet. Man erhält ein Vlies mit einem Flächengewicht von 80 g / m² und einer Dicke von 0.32 mm.

Auf einen Träger (1) werden 5 x 5 mm große Stücke dieses Vlieses (2) aufgeklebt (Figur 3). Mit dem auf den Träger montierten Wischvlies werden die mit Heroin kontaminierten Testfelder mit leichtem Druck abgewischt.

Das auf den Träger montierte Wischvlies wird über dem Konjugatvlies des nach Beispiel 2 b. hergestellten Testträgers positioniert und mit leichtem Druck aufgedrückt und mit einer Klammer fixiert.

Die Elutionsmittelsaufgabezone wird 5 sec. in einen Chromatographiepuffer (150 mmol/l NaCl, 50 mmol/l Kaliumphosphatpuffer pH 7,2) getaucht. Man legt auf eine nicht saugende Unterlage ab, und bestimmt nach 2 min mit einem Chromameter der Fa. Minolta die Buntheit (C-Wert). Danach wird visuell das Nachweisfeld auf die Anwesenheit von rosa Farbe geprüft.

| Menge Heroin Testfeld [ng] | C-Wert | visuelle Ablesung¹ |
|---|---|---|
| 0 | 3.36 | 0 |
| 10 | 2.99 | 0 |
| 10 | 3.05 | 0 |
| 10 | 3.30 | 0 |
| 20 | 7.88 | + |
| 20 | 9.46 | + |
| 20 | 6.89 | +/- |
| 40 | 5.68 | +/- |
| 40 | 8.74 | + |
| 40 | 11.35 | + |
| 60 | 12.00 | ++ |
| 60 | 10.31 | + |
| 60 | 15.05 | ++ |
| 100 | 14.17 | ++ |
| 100 | 10.26 | + |
| 100 | 16.85 | ++ |

| | | |
|---|---|---|
| ¹0: keine Rosafärbung im Nachweisfeld sichtbar | | |
| +/- schwache Farbe nachweisbar | | |
| + Farbe achweisbar | | |
| ++ starke Farbe nachweisbar | | |

### Beispiel 4

Mit einer verdünnten Cocainlösung in Wasser wurden analog Beispiel 3 auf eine Polyethylenfolie Testbereiche mit 5, 10, 25, 50, 75 und 100 ng Cocain aufgetragen. Analog Beispiel 3 werden mit einer Vorrichtung nach Schema 2, die jeweils Testträger nach Beispiel 2 a.zur Bestimmung von Cocain enthält die Testbereiche abgewischt. Die Wischvliese sind rund bei einem Durchmesser von 4 mm. Das Nachweisfeld wird jeweils visuell auf Rosafärbung geprüft.

| Menge Cocain / Testfeld [ng] | visuelle Ablesung² |
|---|---|
| 0 | 0 |
| 5 | +/- |
| 10 | +/- |
| 25 | + |
| 50 | ++ |
| 75 | ++ |
| 100 | ++ |

| | |
|---|---|
| ²O: keine Rosafärbung im Nachweisfeld sichtbar | |
| +/- schwache Farbe nachweisbar | |
| + Farbe achweisbar | |
| ++ starke Farbe nachweisbar | |

### Beispiel 5

Ein Teil Cocain wird mit 1000 Teilen Lactose vermischt. 5 mg dieses Gemisches werden auf einer Fläche von 220 cm² eines Baumwolltuches verteilt. Ca. 10 cm² diese Bereiches werden anaolg Beispiel 4 abgewischt und analysiert. In allen Versuchen wird eine Rosafärbung in dem Nachweisfeld visuell detektiert. Analog wird eine Polyethylenfolie von 2 cm² mit cocainhaltigen Partikeln kontaminiert und durch Wischen analysiert. Es wird ebenfalls in allen Versuchen wird eine Rosafärbung in dem Nachweisfeld visuell detektiert.

### Beispiel 6

Auf eine rauhe anodisierte Aluminiumplatte werden je 10 µl entsprechend verdünnter Lösungen von Cocainhydrochlorid in Wasser aufgegeben und auf eine Fläche von 1 cm² verteilt. Nach Antrocknen bei 37 °C für 15 min. werden die kontaminierten Flächen mit einem runden Vlies nach Beispiel 3 mit einem Durchmesser von 5 mm mit leichtem Druck abgewischt. Dabei wird sowohl ohne Befeuchtung (trocken) als auch nach vorheriger Befeuchtung des Wischvlieses mit 2 µl Wasser gewischt. Die Wischvliese werden dann jeweils auf die Konjugatzone eines Testträgers zum Nachweis auf Cocain nach Beispiel 3 aufgelegt und mit einer flachen Pinzette festgehalten. Das Saugvlies des Testträgers wird zur Hälfte für 10 sec. in Wasser getaucht. Anschließend legt man den Testträger auf einer flachen nicht saugenden Unterlage ab und bestimmte nach 2 min in dem Nachweisfeld die Farbe.

| Absolutmenge Cocainhydrochlorid im Testfeld [ng] | visuelle Ablesung nach Wischen mit trockenem Wischvlies³ | visuelle Ablesung nach Wischen mit feuchtem Wischvlies |
|---|---|---|
| 1000 | ++ | ++ |
| 600 | ++ | ++ |
| 300 | + | ++ |
| 200 | + (schwach) | ++ |
| 100 | 0 | ++ |
| 60 | 0 | ++ |
| 30 | 0 | ++ |
| 25 | 0 | ++ |
| 16 | 0 | ++ |
| 8 | 0 | + |
| 4 | 0 | 0 |

| | | |
|---|---|---|
| ³0: keine Rosafärbung im Nachweisfeld sichtbar | | |
| +: Rosafärbung im Nachweisfeld sichtbar | | |
| ++: starke Rosafärbung im Nachweisfeld sichtbar | | |

## Patentansprüche

1. Verfahren zum Nachweis der Kontamination einer Oberfläche mit einem Analyten durch Abwischen des Analyten von der Oberfläche mit einer Wischfläche, Elution des Analyten von der Wischfläche durch eine Elutionsflüssigkeit und Nachweis des Analyten in der Elutionsflüssigkeit durch eine immunologische Nachweisreaktion, **dadurch gekennzeichnet, daß**
a) mit der Wischfläche die auf den Analyten zu untersuchende Oberfläche abgewischt wird,
b) die Wischfläche mit der flächigen Oberfläche eines kapillaraktiven chromatographiefähigen Materials eines Teststreifens mit einer Elutionsmittelaufgabezone an einem Ende und einer Zielzone am anderen Ende in einem Bereich zwischen beiden Zonen kontaktiert wird,
c) Elutionsflüssigkeit auf die Elutionsmittelaufgabezone aufgegeben wird, die in Richtung zur Zielzone an der Kontaktstelle mit der Wischfläche vorbeiwandert, wobei Analyt von der Elutionsflüssigkeit aufgenommen wird und
d) die Anwesenheit des Analyten in der Zielzone aufgrund einer immunologischen Bindungsreaktion gemessen wird.

2. Verfahren gemäß Anspruch 1, **dadurch gekennzeichnet, daß** der Teststreifen zwischen Elutionsmittelaufgabezone und Zielzone eine Abfangzone enthält, in der ein Abfangreagenz immobilisiert ist, das fähig ist, entweder den Analyten, einen spezifischen Analytbindungspartner oder einen markierten Bindungspartner spezifisch zu binden, und
zwischen der Elutionsmittelaufgabezone und der Abfangzone eine Konjugatzone mit einem wanderungsfähigen markierten Bindungspartner enthält, der fähig ist, entweder den Analyten, einen spezifischen Analytbindungspartner oder das Abfangreagenz spezifisch zu binden, wobei die Bindung des markierten Bindungspartners zu einem detektierbaren Signal in der Abfangzone oder in der Zielzone führt, das die Anwesenheit des Analyten auf der zu untersuchenden Oberfläche anzeigt.

3. Verfahren gemäß Anspruch 2, **dadurch gekennzeichnet, daß** die Wischfläche mit einem Bereich zwischen Elutionsmittelaufgabezone und Konjugatzone kontaktiert wird.

4. Verfahren gemäß Anspruch 2, **dadurch gekennzeichnet, daß** die Wischfläche mit der Konjugatzone selbst kontaktiert wird.

5. Verfahren gemäß Anspruch 2 - 4, **dadurch gekennzeichnet, daß** ein wanderungsfähiger Analytbindungspartner mit einer Bindestelle für das Abfangreagenz zwischen Elutionsmittelaufgabezone und Abfangzone aufgebracht ist.

6. Verfahren gemäß Anspruch 2-4, **dadurch gekennzeichnet, daß** sich in der Konjugatzone ein markierter Bindungspartner für einen Analyten befindet und in der Abfangzone ein immobilisiertes Analyt-Analogon und die Markierung nach Passieren der Abfangzone in der Zielzone als Maß für die Anwesenheit des Analyten detektiert wird.

7. Verfahren gemäß Anspruch 2 - 6, **dadurch gekennzeichnet, daß** der markierte Bindungspartner metallmarkiert ist.

8. Verfahren gemäß Anspruch 1-7, **dadurch gekennzeichnet, daß** es sich beim Analyten um Drogenmoleküle oder Drogenpartikel handelt.

9. Verfahren gemäß Anspruch 2-8, **dadurch gekennzeichnet, daß** das Material der Wischfläche ein Vlies ist.

10. Verfahren gemäß Anspruch 9, **dadurch gekennzeichnet, daß** das Vliesmaterial aus Fasern aus Zellulose und / oder Polyester besteht.

11. Verfahren gemäß Anspruch 1 - 10, **dadurch gekennzeichnet, daß** eine Andrückvorrichtung an dem Teststreifen zum Kontaktieren der Wischfläche mit der Teststreifenoberfläche vorhanden ist.

12. Verfahren gemäß Anspruch 1-11, **dadurch gekennzeichnet, daß** die Wischfläche mit einer Flüssigkeit angefeuchtet ist.

13. Verfahren gemäß Anspruch 12, **dadurch gekennzeichnet, daß** die Wischfläche mit einem wässrigen Puffer angefeuchtet ist.

14. Verfahren gemäß Anspruch 12 oder 13, **dadurch gekennzeichnet, daß** die Flüssigkeit Detergentien und / oder organische Lösungsmittel enthält.

15. Verfahren gemäß Anspruch 12, **dadurch gekennzeichnet, daß** die Wischfläche mit einem organischen Lösungsmittel angefeuchtet ist.

16. Verfahren gemäß Anspruch 12, **dadurch gekennzeichnet, daß** die Wischfläche mit Wasser angefeuchtet ist.

17. Testkit zum Nachweis der Kontamination einer Oberfläche mit einem Analyten durch eine immunologische Nachweisreaktion, **dadurch gekennzeichnet, daß** es umfaßt
a) einen Teststreifen aus einem oder mehreren kapillaraktiven chromatographiefähigen flächenförmigen Materialien in Fluidkontakt zueinander mit
- einer Elutionsmittelaufgabezone an einem Ende und einer Zielzone am anderen Ende,
- einer Abfangzone zwischen Elutionsmittelaufgabezone und Zielzone, in der ein Abfangreagenz immobilisiert ist, das fähig ist, entweder den Analyten, einen spezifischen Analytbindungspartner oder einen markierten Bindungspartner spezifisch zu binden und
- einer Konjugatzone, zwischen Elutionsmittelaufgabezone und Abfangzone, die einen wanderungsfähigen markierten Bindungspartner enthält, der fähig ist, entweder den Analyten oder den spezifischen Analytbindungspartner oder das Abfangreagenz spezifisch zu binden, wobei die Bindung des markierten Bindungspartners zu einem detektierbaren Signal in der Abfangzone oder in der Zielzone führt, das die Anwesenheit des Analyten anzeigt,
b) eine Wischfläche separat zur Teststreifenoberfläche
c) eine Andrückvorrichtung, die bewirkt, daß die Wischoberfläche mit der Teststreifenoberfläche zwischen Elutionsmittelauftragszone und Konjugatzone oder in der Konjugatzone selbst kontaktiert werden kann.

18. Testkit gemäß Anspruch 17, **dadurch gekennzeichnet, daß** in der Konjugatzone ein markierter Bindungspartner für den Analyten sich befindet und in der Abfangzone ein immobilisiertes Analytanalogon sich befindet.

19. Testkit gemäß Anspruch 17 oder 18, **dadurch gekennzeichnet, daß** die Wischfläche aus einem Vliesmaterial besteht.

20. Testkit gemäß Anspruch 19, **dadurch gekennzeichnet, daß** das Vliesmaterial aus Fasern aus Zellulose und / oder Polyester besteht.

21. Testkit gemäß einem der Ansprüche 17 bis 20, **dadurch gekennzeichnet, daß** die Wischfläche mittels Ultraschall mit der Andrückvorrichtung verschweißt ist.

## Claims

1. Method for detecting the contamination of a surface with an analyte by wiping the analyte off the surface with a wiping surface, eluting the analyte from the wiping surface by an elution liquid and detecting the analyte in the elution liquid by an immunological detection reaction, wherein
a) the wiping surface is used to wipe the surface that is to be examined for the analyte,
b) the wiping surface is contacted with the planar surface of a capillary-active chromatographic material of a test strip which has an eluting agent application zone at one end and a target zone at the other end, in an area between the two zones,
c) elution liquid is applied to the eluting agent application zone which moves past the site of contact with the wiping surface during its migration towards the target zone which results in uptake of the analyte by the elution liquid and
d) the presence of the analyte in the target zone is measured by an immunological binding reaction.

2. Method as claimed in claim 1, wherein the test strip contains a capture zone between the eluting agent application zone and target zone in which a capture reagent is immobilized which is able to specifically bind either the analyte, a specific analyte binding partner or a labelled binding partner and
between the eluting agent application zone and the capture zone, contains a conjugate zone containing a labelled binding partner capable of migration which is able to specifically bind either the analyte, a specific analyte binding partner or the capture reagent, the binding of the labelled binding partner resulting in a detectable signal in the capture zone or in the target zone which indicates the presence of the analyte on the surface to be examined.

3. Method as claimed in claim 2, wherein the wiping surface is contacted with an area between the eluting agent application zone and conjugate zone.

4. Method as claimed in claim 2, wherein the wiping surface is contacted with the conjugate zone itself.

5. Method as claimed in claim 2-4, wherein an analyte binding partner capable of migration which has a binding site for the capture reagent is applied between the eluting agent application zone and capture zone.

6. Method as claimed in claim 2-4, wherein a labelled binding partner for an analyte is located in the conjugate zone and an immobilized analyte analogue is located in the capture zone and the label is detected in the target zone after passing the capture zone as a measure for the presence of the analyte.

7. Method as claimed in claim 2-6, wherein the labelled binding partner is labelled with a metal.

8. Method as claimed in claim 1-7, wherein the analyte is a drug molecule or a drug particle.

9. Method as claimed in claim 2-8, wherein the material of the wiping surface is a fleece.

10. Method as claimed in claim 9, wherein the fleece material is composed of fibres made of cellulose and/or polyester.

11. Method as claimed in claim 1-10, wherein the test strip is equipped with a pressing device to contact the wiping surface with the test strip surface.

12. Method as claimed in claim 1-11, wherein the wiping surface is moistened with a liquid.

13. Method as claimed in claim 12, wherein the wiping surface is moistened with an aqueous buffer.

14. Method as claimed in claim 12 or 13, wherein the liquid contains detergents and/or organic solvents.

15. Method as claimed in claim 12, wherein the wiping surface is moistened with an organic solvent.

16. Method as claimed in claim 12, wherein the wiping surface is moistened with water.

17. Test kit for detecting contamination of a surface with an analyte by an immunological detection reaction, wherein it comprises
a) a test strip made of one or several capillary-active chromatographic planiform materials that are in liquid contact with one another and has
- an eluting agent application zone at one end and a target zone at the other end,
- a capture zone between the eluting agent application zone and target zone in which a capture reagent is immobilized that is able to specifically bind either the analyte, a specific analyte binding partner or a labelled binding partner and
- a conjugate zone, between the eluting agent application zone and capture zone, which contains a labelled binding partner capable of migration which is able to specifically bind either the analyte or the specific analyte binding partner or the capture reagent, whereby the binding of the labelled binding partner results in a detectable signal in the capture zone or in the target zone which indicates the presence of the analyte,
b) a wiping surface separate from the test strip surface
c) a pressing device which contacts the wiping surface with the test strip surface between the eluting agent application zone and conjugate zone or in the conjugate zone itself.

18. Test kit as claimed in claim 17, wherein a labelled binding partner for the analyte is located in the conjugate zone and an immobilized analyte analogue is located in the capture zone.

19. Test kit as claimed in claim 17 or 18, wherein the wiping surface is composed of a fleece material.

20. Test kit as claimed in claim 19, wherein the fleece material is composed of fibres made of cellulose and/or polyester.

21. Test kit as claimed in one of the claims 17 to 20, wherein the wiping surface is ultrasonically welded to the pressing device.

## Revendications

1. Procédé pour déceler la contamination d'une surface par un analyte par séparation de l'analyte de la surface par essuyage à l'aide d'une surface d'essuyage, par élution de l'analyte de la surface d'essuyage via un liquide d'élution et par décèlement de l'analyte dans le liquide d'élution via une réaction d'indication immunologique, **caractérisé par** le fait de
a) essuyer, avec la surface d'essuyage, la surface à analyser pour détecter la présence de l'analyte,
b) mettre la surface d'essuyage en contact avec la surface plane d'une matière à activité capillaire apte à une chromatographie d'une bandelette d'essai comprenant une zone de chargement d'éluant à une extrémité et une zone cible à l'autre extrémité à un endroit situé entre les deux zones,
c) charger le liquide d'élution sur la zone de chargement d'éluant, qui migre en direction de la zone cible en passant devant l'endroit de mise en contact avec la surface d'essuyage, l'analyte étant prélevé par le liquide d'élution, et
d) mesurer la présence de l'analyte dans la zone cible sur base d'une réaction de liaison immunologique.

2. Procédé selon la revendication 1, **caractérisé en ce que** la bandelette d'essai contient une zone de fixation entre la zone de chargement de l'éluant et la zone cible dans laquelle un réactif de fixation est immobilisé qui est en mesure de se lier de manière spécifique, soit à un analyte, soit à un partenaire de liaison spécifique à l'analyte, soit à un partenaire de liaison marqué, et contient, entre la zone de chargement de l'éluant et la zone de fixation, une zone de conjugué comprenant un partenaire de liaison marqué manifestant une aptitude à la migration qui est en mesure de se lier de manière spécifique, soit à un analyte, soit à un partenaire de liaison spécifique à l'analyte, soit à un partenaire de liaison marqué, la liaison du partenaire de liaison marqué menant à un signal détectable dans la zone de fixation ou dans la zone cible indiquant la présence de l'analyte sur la surface à analyser.

3. Procédé selon la revendication 2, **caractérisé en ce qu'**on met la surface d'essuyage en contact avec un endroit situé entre la zone de chargement de l'éluant et la zone de conjugué.

4. Procédé selon la revendication 2, **caractérisé en ce qu'**on met la surface d'essuyage en contact avec la zone de conjugué elle-même.

5. Procédé selon l'une quelconque des revendications 2 à 4, **caractérisé en ce qu'**on applique entre la zone de chargement de l'éluant et la zone de fixation, un partenaire de liaison à l'analyte manifestant une aptitude à la migration, comprenant un endroit de liaison pour le réactif de fixation.

6. Procédé selon l'une quelconque des revendications 2 à 4, **caractérisé en ce qu'**un partenaire de liaison marqué pour un analyte est disposé dans la zone de conjugué et un analogue d'analyte immobilisé est disposé dans la zone de fixation, le marquage, après le passage de la zone de fixation dans la zone cible, étant détecté comme mesure pour la présence de l'analyte.

7. Procédé selon l'une quelconque des revendications 2 à 6, **caractérisé en ce que** le partenaire de liaison marqué est soumis à un marquage à l'aide d'un métal.

8. Procédé selon l'une quelconque des revendications 1 à 7, **caractérisé en ce qu'**il s'agit, en ce qui concerne l'analyte, de molécules de drogues ou de particules de drogues.

9. Procédé selon l'une quelconque des revendications 2 à 8, **caractérisé en ce que** la matière de la surface d'essuyage est un non-tissé.

10. Procédé selon la revendication 9, **caractérisé en ce que** le non-tissé est constitué par des fibres de cellulose et/ou de polyester.

11. Procédé selon l'une quelconque des revendications 1 à 10, **caractérisé en ce qu'**on prévoit un dispositif d'application par pression sur la bandelette d'essai pour la mise en contact de la surface d'essuyage avec la surface de la bandelette d'essai.

12. Procédé selon l'une quelconque des revendications 1 à 11, **caractérisé en ce que** la surface d'essuyage est légèrement humidifiée avec un liquide.

13. Procédé selon la revendication 12, **caractérisé en ce que** la surface d'essuyage est légèrement humidifiée avec un tampon aqueux.

14. Procédé selon la revendication 12 ou 13, **caractérisé en ce que** le liquide contient des détergents et/ou des solvants organiques.

15. Procédé selon la revendication 12, **caractérisé en ce que** la surface d'essuyage est légèrement humidifiée avec un solvant organique.

16. Procédé selon la revendication 12, **caractérisé en ce que** la surface d'essuyage est légèrement humidifiée avec de l'eau.

17. Nécessaire d'essai pour déceler la contamination d'une surface par un analyte via une réaction d'indication immunologique, **caractérisé en ce qu'**il comprend
a) une bandelette d'essai constituée par une ou plusieurs matières de forme plate à activité capillaire et aptes à une chromatographie mises en contact réciproque par fluide comprenant
- une zone de chargement d'éluant à une extrémité et une zone cible à l'autre extrémité,
- une zone de fixation entre la zone de chargement d'éluant et la zone cible, dans laquelle est immobilisé un réactif de fixation qui est en mesure de se lier de manière spécifique, soit à un analyte, soit à un partenaire de liaison spécifique à l'analyte, soit à un partenaire de liaison marqué, et
- une zone de conjugué, entre la zone de chargement de l'éluant et la zone de fixation, qui contient un partenaire de liaison marqué manifestant une aptitude à la migration qui est en mesure de se lier de manière spécifique, soit à un analyte, soit à un partenaire de liaison spécifique à l'analyte, soit à un partenaire de liaison marqué, la liaison du partenaire de liaison marqué menant à un signal détectable dans la zone de fixation ou dans la zone cible qui indique la présence de l'analyte,
b) une surface d'essuyage séparée de la surface de la bandelette d'essai,
c) un dispositif d'application par pression qui fait en sorte que l'on peut mettre la surface d'essuyage en contact avec la surface de la bandelette d'essai entre la zone de chargement de l'éluant et la zone de conjugué ou dans la zone de conjugué elle-même.

18. Nécessaire d'essai selon la revendication 17, **caractérisé en ce qu'**un partenaire de liaison marqué pour l'analyte est disposé dans la zone de conjugué et un analogue d'analyte immobilisé est disposé dans la zone de fixation.

19. Nécessaire d'essai selon la revendication 17 ou 18, **caractérisé en ce que** la matière de la surface d'essuyage est un non-tissé.

20. Nécessaire d'essai selon la revendication 19, **caractérisé en ce que** le non-tissé est constitué par des fibres de cellulose et/ou de polyester.

21. Nécessaire d'essai selon l'une quelconque des revendications 17 à 20, **caractérisé en ce que** la surface d'essuyage est soudée par ultrasons au dispositif d'application par pression.
